# EUROPEAN PATENT APPLICATION

(11) **EP 0 669 104 A1**
(43) Date of publication of application: **30.08.1995**
(21) Application number: 95301212.7
(22) Date of filing: 24.02.1995
(51) Int. Cl.: A61B 17/072

(54) **Anvil pockets for surgical stapler**

(30) Priority: 25.02.1994 US 201845
(71) Applicant: ETHICON ENDO-SURGERY, Cincinnati, Ohio 45242-2839 (US)
(72) Inventor: Bittner, John R., Loveland, Ohio 45140 (US); Burrington, Elmer E., Cincinnati, Ohio 45247 (US); Main, Lauren O., Loveland, Ohio 45140 (US); Welch, Robert F., Maineville, Ohio 45039 (US); Yeager, Robert H., Twinsburg, Ohio 44087 (US)
(74) Representative: Fisher, Adrian John

(57) **Abstract**

In accordance with the invention, a surgical stapling instrument comprises first and second cooperating elongate jaw members one of the jaw members including stapling carrying means adapted to receive a plurality of staples arranged in at least one row, and the other jaw member including adapted and improved anvil means adapted to form the staples, pusher means for driving the staples from the staple carrying means into tissue gripped between the jaw members and forming the staples against the anvil means to produce at least one row of staples in the tissue, knife means for cutting the tissue gripped between the jaw members along a line adjacent to the row of staples, and jaw clamping means for applying clamping forces to the jaw members to resist the forces exerted on the staple carrying means and anvil means when the staples are formed. The improved anvil means are able to more readily form the staples, and comprise a skewed surface, either in the lateral or depth direction, which results in the points of the staple always missing one another upon forming.

## Description

### FIELD OF THE INVENTION

The present invention relates to a surgical stapling instrument and, more particularly, to a gastrointestinal anastomotic stapling instrument for producing one or more rows of staples when the staples are formed.

### BACKGROUND OF THE INVENTION

In recent years, there has been an increasing tendency for surgeons to use stapling instruments to suture body organs and tissues such as lung, esophagus, stomach, duodenum and other body organs in the intestinal tract. The use of an appropriate stapling instrument in most instances performs a better job in less time and simplifies previously difficult surgical procedures such as gastrointestinal anastomoses.

Typically, a linear anastomotic stapling instrument includes a pair of cooperating elongate jaw members, each adapted to be inserted into internal, tubular body organs to be anastomosed. One of the jaw members supports a staple cartridge with at least two laterally spaced rows of staples, and the other jaw member supports an anvil with staple-forming pockets aligned with the rows of staples in the cartridge. Generally, a single pusher bar and knife assembly is slidable longitudinally along the jaw members to sequentially eject staples from the cartridge via camming surfaces which activate a plurality of staple drivers carried by the cartridge and associated with the individual staples to close the staples against the anvil and form laterally spaced rows of staples in the tissue gripped between the jaw members. A knife blade which trails the pusher bars cuts the tissue along a line between the staple rows. Yet, no provision is made in typical staplers for the points of the stapler contacting one another in the anvil pockets.

### SUMMARY OF THE INVENTION

In accordance with the invention, a surgical stapling instrument comprises first and second cooperating elongate jaw members one of the jaw members including stapling carrying means adapted to receive a plurality of staples arranged in at least one row, and the other jaw member including adapted and improved anvil means adapted to form the staples, pusher means for driving the staples from the staple carrying means into tissue gripped between the jaw members and forming the staples against the anvil means to produce at least one row of staples in the tissue, knife means for cutting the tissue gripped between the jaw members along a line adjacent to the row of staples, and jaw clamping means for applying clamping forces to the jaw members to resist the forces exerted on the staple carrying means and anvil means when the staples are formed. The improved anvil means are able to more readily form the staples, and comprise a skewed surface, either in the lateral or depth direction, which results in the points of the staple always missing one another upon forming.

### BRIEF DESCRIPTION OF THE DRAWINGS

Figure 1 is an overall perspective view of a linear anastomotic stapling instrument embodying the principles of the present invention;
Figure 2 is a side elevation showing the anastomotic stapling instrument partially disassembled with its upper anvil carrying jaw member detached from its lower staple cartridge carrying jaw member;
Figure 3 is a side elevation showing the anastomotic stapling instrument in its assembled configuration;
Figure 4 is a side elevation, partially in section, of the anastomotic stapling instrument showing a cam mechanism for urging the rear portions of the upper and lower jaw members apart;
Figure 5 is a bottom view of the anvil carrying jaw member of the anastomotic stapling instrument;
Figure 6 is a top view of the staple cartridge carrying jaw member of the anastomotic stapling instrument;
Figure 7 is a bottom view of the anastomotic stapling instrument;
Figure 8 is a front end view of the anastomotic stapling instrument;
Figure 9 is a rear end view of the anastomotic stapling instrument;
Figure 10 is an enlarged perspective view of a pusher bar and knife blade assembly of the anastomotic stapling instrument;
Figure 11 is an enlarged perspective view of a pusher block and an actuator knob which are components of the pusher bar and knife blade assembly of the anastomotic stapling instrument;
Figure 12 is an enlarged elevation, partially in section, of the rear portion of the anastomotic stapling instrument illustrating the cam mechanism in its inoperative position;
Figure 13 is an enlarged elevation, partially in section, of the rear portion of the anastomotic stapling instrument illustrating the cam mechanism in its operative position;
Figure 14 is an enlarged side view of the staple cartridge of the anastomotic stapling instrument;
Figure 15 is an enlarged top view of the staple cartridge of the anastomotic stapling instrument;
Figure 16 is an enlarged bottom view of the staple cartridge of the anastomotic stapling instrument;
Figure 17 is an enlarged, partially cutaway view of the anvil and staple cartridge carrying jaw members of the anastomotic stapling instrument;
Figure 18 is an enlarged, partially cutaway view of the anvil and staple cartridge carrying jaw members illustrating the operation of the pusher bar and knife blade assembly;
Figure 19 is an enlarged vertical section of the anastomotic stapling instrument taken along line 19-19 of Figure 4;
Figure 20 is an enlarged vertical section of the anastomotic stapling instrument taken along line 20-20 of Figure 4;
Figure 21 is an enlarged section of a portion of the anvil and staple cartridge shown in Figure 18;
Figures 22 and 22a are views of anvil pockets able to form a single staple;
Figure 23 is a view of an alternate embodiment of the anvil pocket configuration of Figure 22; and
Figures 24, 24(a), 24(b), 25, 25(c) and 25(d) are alternate configurations of anvil pockets embodying the present invention.

### DETAILED DESCRIPTION OF THE INVENTION

There is described in this invention a particularly modified anvil for forming surgical staples. Of course, it is to be realized that while the embodiment disclosed herein is a linear stapler, any type stapler is possible to use with this invention. Thus, the stapler may be circular in cross section, apply a single row of staples, or any other conceivable embodiments without departing from the scope of this invention.

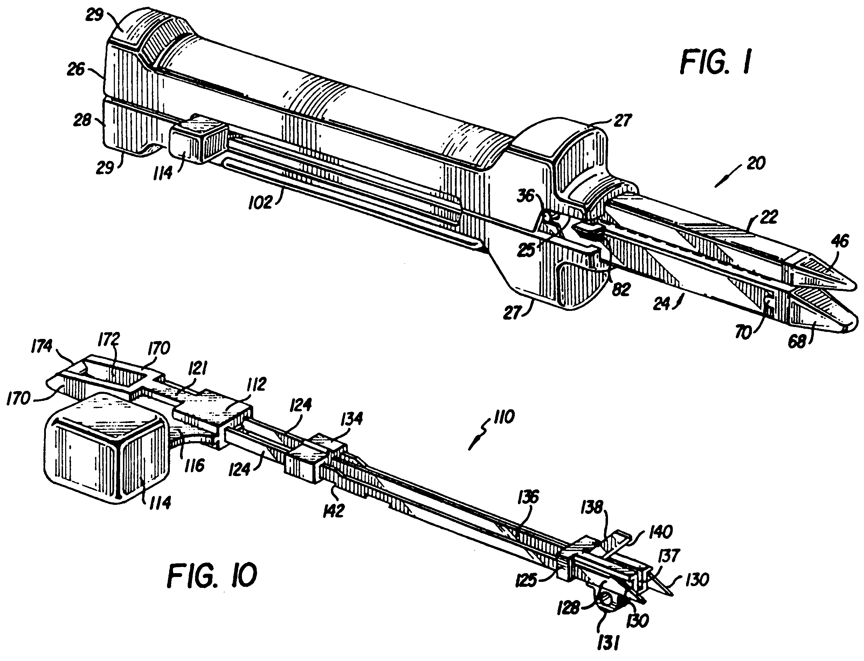

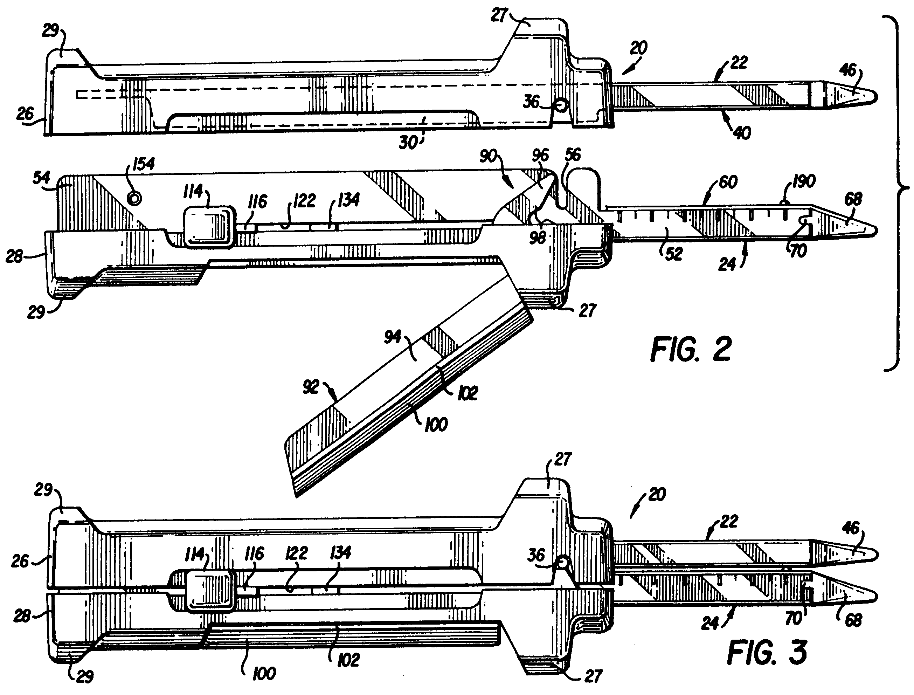

Referring to Figures 1 and 2, the present invention is embodied in a linear anastomotic stapling instrument, generally 20, comprising an upper elongated anvil carrying jaw member 22 and a lower elongated staple cartridge carrying jaw member 24. Upper anvil carrying jaw member 22 is supported by a handle 26 with a front portion of the jaw member extending forwardly therefrom. Lower staple cartridge carrying jaw member 24 is supported by a handle 28 with a front portion of the jaw member extending forwardly therefrom. As shown in Figure 3, upper handle 26 and lower handle 28 are suitably shaped to form a hand grip to facilitate the handling and operation of the stapling instrument by a surgeon. An enlarged front protrusion 27 and a small rear protrusion 29 are provided on each handle for this purpose. Preferably, handles 26 and 28 are made of plastic of other lightweight material, while jaw members 22 and 24 are made of stainless steel or other similar material.

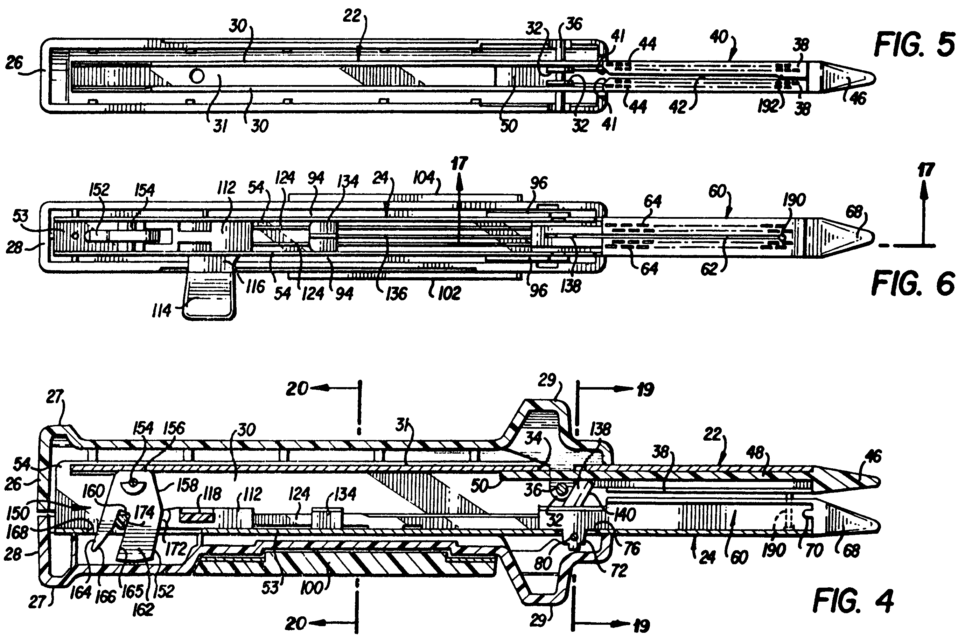

As shown in Figure 5, upper jaw member 22 comprises a one-piece elongated channel-shaped frame including a pair of opposed, elongated side walls 30 connected by a top wall 31. Upper handle 26 includes a pair of depending ears 32 located inside the upper handle adjacent to its front end. Upper jaw member 22 includes a slot 34 (Figure 4) formed at an intermediate position along its top wall 31 through which depending ears 32 project downward. A latch pin 36 extends through circular holes formed in side walls 30 of upper jaw member 22 and through circular holes formed in depending ears 32 to pivotally connect the upper jaw member to upper handle 26.

Referring to Figure 5, the front portion of upper jaw member 22 is provided with a pair of elongated inwardly extending flanges 38 which define an anvil 40 of the stapling instrument. Flanges 38 are separated by a central longitudinal slot 42 which extends along the entire length of anvil 40. At the proximal end of central slot 42, the flanges 38 are provided with inwardly sloped guide surfaces 41. Each flange 38 is also provided with two longitudinal rows of uniformly spaced staple-forming pockets 44.

Referring to Figures 4 and 5, a tapered anvil tip 46 is mounted at the front of anvil carrying jaw member 22 to facilitate the insertion of the jaw member into hollow, tubular body organs. Anvil tip 46 includes an elongated body 48 (Figure 4) which is inserted through the longitudinal passageway above anvil 40 defined by side walls 30 and flanges 38 of the upper jaw member. This elongated body 48 extends between depending ears 32 above latch pin 36 and includes an enlarged rear portion 50 located behind ears 32 to hold anvil tip 46 in place on upper jaw member 22.

Referring to Figures 2 and 6, lower cartridge carrying jaw member 24 comprises a one-piece elongated channel-shaped frame including a pair of opposed, elongated side walls 52 connected by a bottom wall 53. Along the rearward portion of lower jaw member 24, a pair of spaced, elongated upstanding side flanges 54 (Figure 2) extend upward from its opposed side walls 52. As shown in Figures 5 and 6, the width of lower jaw member 24 between its side flanges 54 is greater than the width of upper jaw member 22 between its side walls 30 to permit the rear portion of the upper jaw member to be received between side flanges 54 of the lower jaw member when the stapling instrument is assembled for operation. As shown in Figure 2, each side flange 54 of lower jaw member 24 includes a vertical notch 56 located in alignment with latch pin 36 on upper jaw member 22. When upper jaw member 22 and lower jaw member 24 are assembled, the opposite ends of latch pin 36 are received in notches 56.

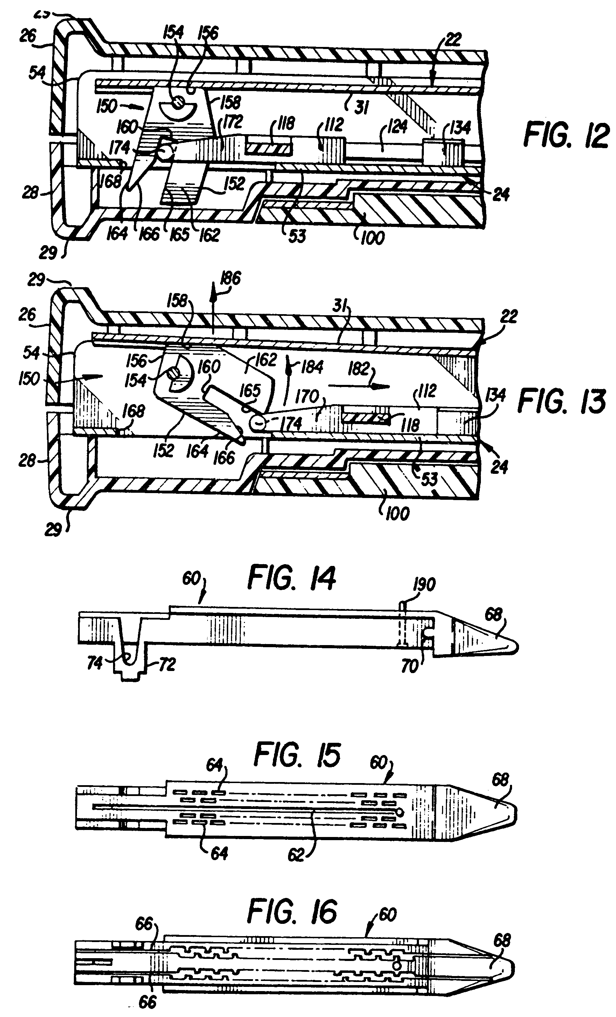

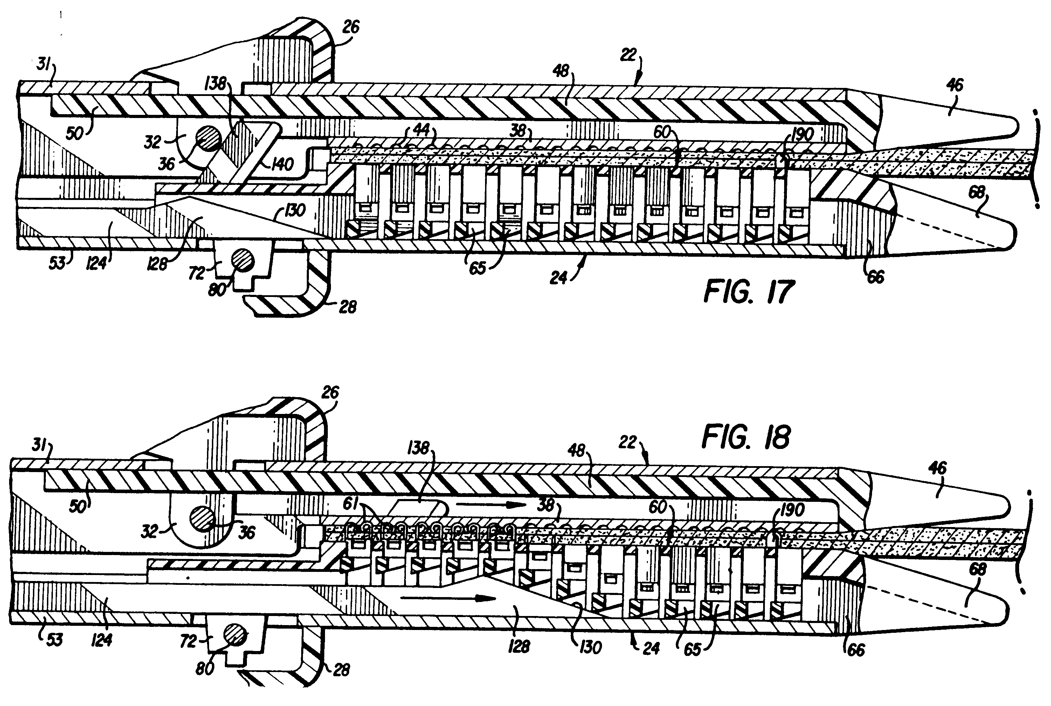

As shown in Figures 2 and 6, lower jaw member 24 supports a staple cartridge 60 which is adapted to receive a plurality of surgical staples 61 (Figure 18) arranged in at least two laterally spaced longitudinal rows. Staple cartridge 60 is mounted at the front portion of lower jaw member 24 between its side walls 52. Staple cartridge 60 is divided longitudinally by a central, elongated slot 62 (Figure 6) which extends from the proximal end of the cartridge toward its distal end. Preferably, a plurality of staple openings 64 formed in staple cartridge 60 is arranged in two pairs of laterally spaced rows, with each pair of rows disposed on opposite sides of central longitudinal slot 62. A plurality of surgical staples 61 (Figure 18) are mounted within openings 64 of cartridge 60. As shown in Figure 6, the staple openings 64 in adjacent rows are staggered to provide more effective stapling of the tissue when the instrument is operated. Referring to Figures 15 and 16, staple cartridge 60 includes a pair of longitudinal slots 66 located on opposite sides of elongated central slot 62 and disposed between the staggered rows of openings 64 on each side of the central slot. Each longitudinal slot 66 extends from the proximal end of cartridge 60 towards its distal end.

As shown in Figure 17, a plurality of staple drivers 65 is slidably mounted in staple openings 64 for actuating the staples 61 which are loaded into staple cartridge 60. Referring to Figure 6, each staple driver 65 is designed to simultaneously actuate two staples 61 located in the adjacent rows provided in staple cartridge 60. Thus, a first set of staple drivers 65 is provided for actuating the staples 61 in the staggered rows located on one side of central longitudinal slot 62, and a second set of staple drivers 65 is provided for actuating the staples 61 in the pair of adjacent rows located on the other side of central longitudinal slot 62.

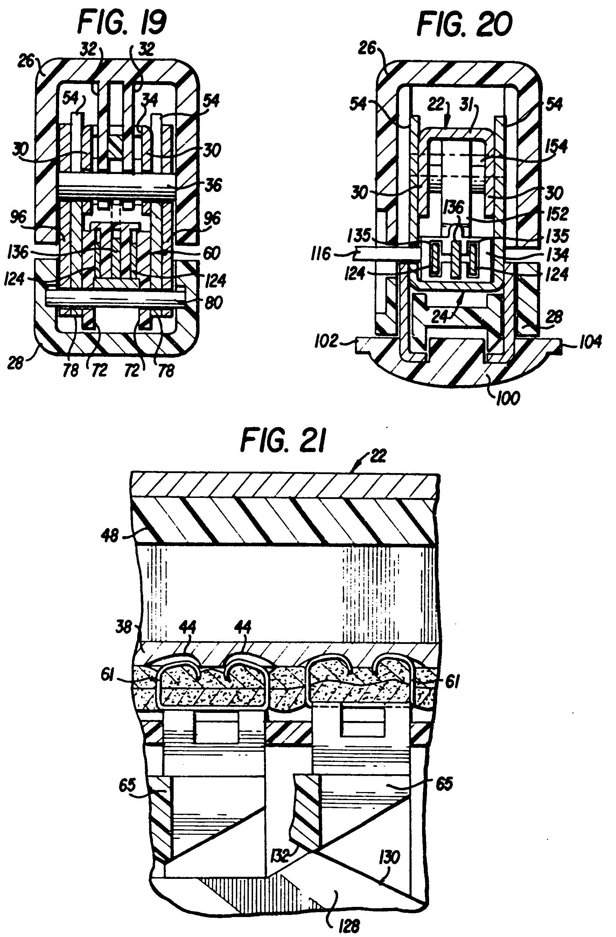

As shown in Figures 2 and 3, the front or distal end of staple cartridge 60 includes a tapered tip 68 to facilitate the insertion of lower jaw member 24 into a hollow, tubular body organ. Immediately behind its tapered tip 68, staple cartridge 60 is provided with a pair of rearwardly extending protrusions 70 (one shown in Figure 14) which re received in corresponding notches provided in side walls 52 of lower jaw member 24. At the rear of staple cartridge 60, a pair of depending arms 72 extends downwardly from the cartridge. Each arm 72 is notched to provide a side opening 74. When cartridge 60 is assembled on lower jaw member 24, its protrusions 70 are received in corresponding notches provided at the front ends of side walls 52 and its depending arms 72 extend downwardly through an opening 76 (Figure 4) formed in bottom wall 53 of jaw member 24. Lower jaw member 24 includes a pair of depending ears 78 (Figure 19) extending downwardly from its side walls 52 on opposite sides of opening 76. A pivot pin 80 extends through holes formed in depending ears 78 of lower jaw member 24 and through side openings 74 of lower jaw member 24 and through side openings 74 of depending arms 72 on staple cartridge 60 to fasten the staple cartridge to the lower jaw member.

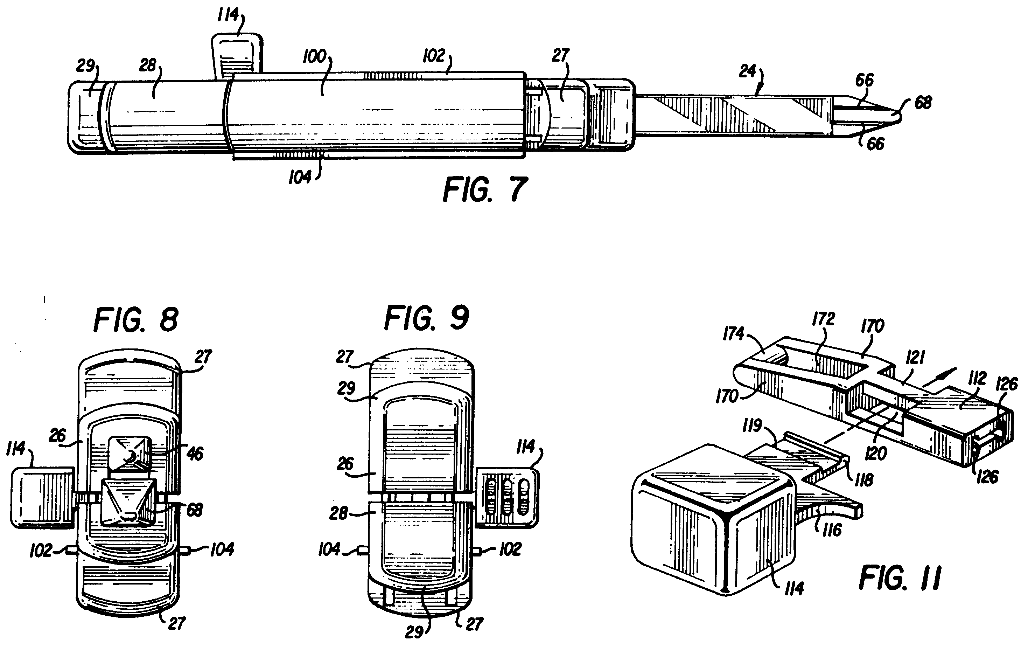

Referring to Figure 2, the stapling instrument 20 includes a latching mechanism, generally 90, for latching upper jaw member 22 and lower jaw member 24 together at an intermediate position along the jaw members. Preferably, jaw members 22 and 24 are latched together at a position adjacent to the proximal ends of anvil 40 and staple cartridge 60. In the preferred embodiment, latching mechanism 90 comprises a latch arm 92 (Figure 2) pivotally connected to lower jaw member 24 via pivot pin 80 (Figure 4). Latch arm 92 is channel-shaped in configuration and includes a pair of opposed, elongated side walls 90 (Figure 6) which are spaced apart by a distance sufficient to span side walls 52 of lower jaw member 24. Each side wall 94 of latch arm 92 includes an upwardly and forwardly extending hook member 96 provided with a forwardly facing slot 98 for receiving latch pin 36. A shroud 100 is mounted on the lower surface of latch arm 92. When latch arm 92 is closed, as shown in Figure 3, shroud 100 is aligned with the bottom of lower handle 28 to facilitate the handling and operation of stapling instrument 20 by the surgeon. Preferably, shroud 100 is made of plastic or other light-weight material, while latch arm 92 is made of stainless steel. As shown in Figure 7, shroud 100 includes elongated flanges 102 and 104 extending outwardly from its opposite sides which serve as finger grips to enable latch arm 92 to be pivoted downward from its latched to it unlatched position. When latch arm 92 is moved to its closed or latched position, the surfaces of slots 98 of hook members 96 cooperate with latch pin 36, acting as an over-center latch to maintain latch arm 92 in its latched position.

Referring to Figures 6 and 10, the preferred embodiment of stapling instrument 20 includes an improved pusher bar and knife blade assembly, generally 110, which is slidably mounted for longitudinal movement relative to upper and lower jaw members 22 and 24, respectively, for driving staples 61 from staple cartridge 60 into tissue gripped between the jaw members, forming staples 61 against anvil 40, and cutting the tissue along a line between the rows of staples formed in the tissue. Pusher bar and knife blade assembly 110 includes a pusher block 112 (Figure 6) which is slidably received within the lower channel-shaped jaw member 24 between its upstanding side flanges 54. As shown in Figure 11, pusher block 112 is attached to an actuator knob 114 by a flange 116 which includes a laterally projecting finger 118 provided with a longitudinally extending notch 119 on its top surface. Finger 118 is snap-fitted into a lateral slot 120 formed in pusher block 112 to locate notch 119 underneath a longitudinal locking bar 121 to secure pusher block 112 and actuator knob 114 together. Flange 116 of actuator knob 114 extends through and rides along an elongated slot 122 (Figure 2) formed in one side flange 54 of lower jaw member 24.

The pusher bar and knife blade assembly 110 includes a pair of staple pusher bars 124 (Figure 10) projecting forwardly from pusher block 112 and slidably received in elongated slots 66 (Figure 16) of staple cartridge 60. Pusher block 112 is provided with a pair of vertical slots 126 (Figure 11) in which pusher bars 124 are secured. As shown in Figure 10, the front end of each staple pusher bar 124 is provided with a wedge-shaped tip 128 which defines an inclined cam surface 130 for engaging staple drivers 65 as pusher bars 124 are advanced into staple cartridge 60. As shown in Figure 21, each staple driver 65 is provided with a sloped surface 132 oriented at the same angle as cam surface 130 of each staple pusher bar 124 to provide a flat, sliding contact between the surfaces.

Referring to Figures 6 and 10, the pusher bar and knife blade assembly 110 includes a knife block 134 which is slidably mounted for longitudinal movement along lower jaw member 24 between it upstanding side flanges 54. Knife block 134 includes a knife support bar 136 which extends forwardly into central longitudinal slot 62 of staple cartridge 60. An inclined knife blade 138 is provided with a beveled cutting edge 140 is located at the front end of knife support bar 136. The beveled cutting edge of knife blade 138 is oriented at an angle relative to elongate jaw members 22 and 24 and is slidably received in central longitudinal slot 62 of staple cartridge 60.

In the preferred embodiment of stapling instrument 20, knife block 134 includes a pair of longitudinal slots 135 (Figure 20) extending therethrough which slidably receive staple pusher bars 124 to permit pusher block 112 to slide relative to the knife block. Accordingly, when pusher block 112 is advanced toward staple cartridge 60 by actuator knob 114, staple pusher bars 124 slide through knife block 134 which remains stationary until the pusher block moves into engagement with the knife block. After knife block 134 is engaged by pusher block 112, the knife block and pusher block advance simultaneously toward staple cartridge 60. As shown in Figure 18, knife blade 138 is advanced through staple cartridge 60 along with staple pusher bars 124, forming staples 61 in the tissue gripped between the jaw members and cutting the tissue between the staple rows. Thereafter, when actuator knob 114 is retracted, pusher block 112 initially slides staple pusher bars 124 backward through knife block 134 which remains stationary. Each staple pusher bar 124 includes an offset portion 142 which moves into engagement with knife block 134 after staple pusher bars 124 are withdrawn by a predetermined distance. With offset portions 142 of staple pusher bars 124 engaging knife block 134, pusher block 112 and knife block 134 are simultaneously retracted by actuator knob 114 to return pusher bars 124 and knife blade 138 to the start position.

In accordance with the invention, stapling instrument 20 is provided with jaw clamping means for applying clamping forces to the jaw members to urge staple cartridge 60 and anvil 40 together during the formation of staples 61. The jaw clamping means includes means for urging the jaw members apart at a position remote from the latching mechanism to resist the forces exerted on staple cartridge 60 and anvil 40 when staples 61 are formed. In the preferred embodiment, a cam means is mounted on one of the jaw members and engageable with the other jaw member for moving said jaw members apart the remote position to urge staple cartridge 60 and anvil 40 together. Preferably, a cam member is pivotally mounted on one of the jaw members at a position remote from the latching mechanism. The cam member is pivotable from a first inoperative position to a second operative position to move the remote ends of the jaw members apart. The cam member is operable by pusher block 112 of pusher bar and knife blade assembly 110 to more to its operative position when the pusher block is advanced and to return to its inoperative position when the pusher block is retracted.

In the preferred embodiment of the stapling instrument 20, a cam mechanism, generally 150, is located adjacent to the rear end of lower jaw member 24, as shown in Figure 4. Cam mechanism 150 includes a cam member 152 pivotally mounted on a transverse pivot pin extending between upstanding side flanges 54 of lower jaw member 24. Cam member 152 includes a first lower cam surface 156 for engaging top wall 31 of upper jaw member 22 with cam 152 in its first inoperative position (Figure 12) and a second higher cam surface 158 for engaging the top wall 31 of upper jaw member 22 with cam 152 disposed in its second operative position (Figure 13). First cam surface 156 is arranged to maintain upper and lower jaw members substantially parallel with cam 152 in its inoperative position. Second cam surface 158 is arranged to raise the rear end of upper jaw member 22 by approximately 0.125 inch (3.2mm) when cam 152 pivots from its inoperative position to its operative position. In addition, upper jaw member 22 is sufficiently flexible to permit the rear portion of upper jaw member 22 to bend upward away from lower jaw member 24 when cam member 152 is moved from its inoperative position to its operative position.

As shown in Figure 4, cam member 152 includes a radially extending notch 160 which divides the cam into a large front finger 162 and a small rear finger 164. Front cam finger 162 includes a flat, rearwardly facing surface 165, and rear cam finger 164 includes a sloped, forwardly facing surface 166. With cam 152 in its inoperative position, front cam finger 162 and rear cam finger 164 extend downwardly through an elongated slot 168 formed in bottom wall 53 of lower jaw member 24.

In the preferred embodiment, cam member 152 is operable by pusher block 112 to move from its inoperative position to its operative position when the pusher block is advanced. As shown in Figure 11, pusher block 112 includes a pair of rearwardly extending arms 170 which are spaced apart to define a gap 172 therebetween. The rear ends of arms 170 are connected by a cam actuator pin 174 which extends across gap 172. Referring to Figures 4 and 11, with cam member 152 disposed in its inoperative position, front cam finger 162 extends through gap 172 between arms 170 of pusher block 112, while cam actuator pin 174 is received in notch 160 between front finger 162 and rear finger 164 of the cam member.

As shown in Figure 12, with cam member 152 disposed in its first inoperative position, top wall 31 of upper jaw member 22 rests on first cam surface 156 of the cam member. With cam member 152 in its inoperative position, top wall 31 of upper jaw member 22 is substantially parallel to bottom wall 53 of lower jaw member 24. In addition, pusher block 112 is located in its start position spaced rearwardly from knife block 134. When pusher block 112 is advanced, as indicated by arrow 182 (Figure 13), cam actuator pin 174 engages rear surface 165 of front cam finger 162 to rotate cam member 152 in a counter-clockwise direction, as indicated by arrow 184, to pivot the cam member to its second operative position and move its second cam surface 158 into engagement with top wall 31 of upper jaw member 22. With cam member 152 pivoted to its operative position, the top wall 31 of upper jaw member 22 is bent upwardly, as indicated by arrow 186, away from bottom wall 53 of lower jaw member 24. The cam member applies forces to upper jaw member 22 and lower jaw member 24 which bend the rear portions of jaw members apart. As a result of the bending the rear portions of upper jaw member 22 and lower jaw member 24 apart, additional clamping forces are applied to the front portions of upper jaw member 22 and lower jaw member 24 to clamp anvil 40 and staple cartridge 60 against the tissue gripped between the jaw members. Thus, anvil 40 and staple cartridge 60 are urged together to resist the forces exerted on the anvil and staple cartridge when pusher bar and knife blade assembly 110 is advanced to form staples 61 and cut the tissue.

Referring to Figure 13, when pusher block 112 is retracted after staples 61 are formed, cam actuator pin 174 engages sloped surface 166 of rear cam finger 164 to pivot cam member 152 in a clockwise direction. As cam actuator pin 174 moves along sloped surface 166 into notch 160, cam member 152 pivots in a clockwise direction and returns to its first inoperative position (Figure 12) with its first cam surface 156 in engagement with top wall 31 of upper jaw member 22. As a result, the forces exerted on the rear portions of upper jaw member 22 and lower jaw member 24 by cam 152 are released and top wall 31 of upper jaw member 22 returns to a substantially parallel relationship with bottom wall 53 of lower jaw member 24. Similarly, the clamping forces applied to the front portions of jaw members 22 and 24 are released to unclamp anvil 40 and staple cartridge 60.

The preferred embodiment of stapling instrument 20 includes spacer means mounted on one of the jaw members for maintaining a predetermined gap between staple cartridge 60 and anvil 40 of the stapling instrument. Referring to Figures 4 and 6, this spacer means is embodied as spacer pin 190 mounted adjacent to the distal end of staple cartridge 60. Spacer pin 190 extends vertically upward from bottom wall 53 of lower jaw member 24 through staple cartridge 60 and projects upwardly from the top of the staple cartridge by a predetermined distance. As shown in Figure 5, one flange 38 of anvil 40 includes a flange section 192 adjacent to its distal end for engaging spacer pin 190. With the stapling instrument assembled for operation (Figure 4), spacer pin 190 engages flange section 192 to maintain a predetermined gap between anvil 40 and staple cartridge 60.

In the operation of stapling instrument 20, the tissue to be stapled and cut must be initially placed between jaw members 22 and 24 and clamped by the jaw members. Thus, handles 26 and 28 are unlatched by pivotal movement of latch arm 92 downward to its unlatched position (Figure 2). As a result, the opposite ends of latch pin 36 are disengaged from slots 98 formed in hook member 96 of latching arm 92. Thereafter, upper and lower jaw members 22 and 24 can be separated by disengaging latch pin 36 from slots 56 formed in side flanges 54 of the lower jaw member.

Next, the tissue to be stapled and cut is placed on jaw members 22 and 24. For example, as shown in Figure 17, a piece of tubular, intestinal tissue may be slipped onto the front portion of each jaw member. After the tissue is placed on the jaw member, stapling instrument 20 is reassembled. The reassembly can be accomplished by aligning latch pin 36 with vertical slots 56 formed in upstanding side flanges 54 of lower jaw member 24. Thereafter, side flanges 54 of lower jaw member 24 are positioned inside upper handle 26, spanning side walls 30 of upper jaw member 22, while the opposite ends of latch pin 36 are inserted into vertical slots 56. Finally, latch arm 92 is pivoted upward to its latched position (Figure 3) with its cover 100 flush with the bottom of lower handle 28. As a result, hook members 92 are pivoted over latch pin 36 and slots 98 receive the opposite ends of the latch pin. Thus, upper jaw member 22 and lower jaw member 24 are latched together at an intermediate position there along adjacent to anvil 40 and staple cartridge 60. In addition, spacer pin 190 engages flange section 192 of anvil 40 through the body tissue to maintain a predetermined gap between anvil 40 and staple cartridge 60.

After the tissue is clamped between the jaw members, stapling instrument 20 is fired by advancing actuator knob 114 to actuate the pusher bar and knife blade assembly 110. Initially, in the actuation of cam mechanism 150, pusher block 112 and pusher bars 124 (Figure 4) are advanced, while knife block 134 remains stationary. Since only pusher block 112 and its pusher bars 124 are advanced to actuate cam member 152, the initial force required to operate stapling instrument 20 is minimized.

Referring to Figure 12, during the initial advance of pusher block 112, pusher bars 124 slide through knife block 134 and the wedge-shaped tips 128 of the pusher bars begin to advance through slots 66 of staple cartridge 60. As pusher block 112 advances toward knife block 134, its cam actuator pin 174 engages rear surface 165 of front cam finger 162 to pivot cam 152 counter-clockwise, as indicated by arrow 184 of Figure 13, to move the second cam surface 158 of the cam member into engagement with top wall 31 of upper jaw member 22. Cam member 152 applies forces to upper jaw member 22 and lower jaw member 24 which bend the rear portions of the jaw members apart. As a result, the rear end of top wall 31 of upper jaw member 222 is bent upward by approximately 0.125 inch (3.2 mm) relative to the rear end of bottom wall 53 of lower jaw member 24. The bending of the rear ends of jaw members 22 and 24 apart results in additional clamping forces on the front portions of the jaw members to clamp anvil 40 and staple cartridge 60 against the tissue gripped between the jaw members. These additional clamping forces tend to resist the fores exerted on anvil 40 and staple cartridge 60, while the tissue is cut and staples 61 are formed against anvil 40, to maintain the desired spacing between anvil 40 and staple cartridge 60 to produce formed staples 61 which are substantially uniform in height.

Referring to Figure 13, after cam mechanism 150 is actuated, pusher block 112 subsequently engages knife block 134 to begin the longitudinal movement of knife block 134 toward staple cartridge 60. Preferably, the initial spacing between pusher block 112 and knife block 134 is arranged such that pusher block 112 engages knife block 134 slightly before cam member 152 arrives at its operative position. Alternatively, the initial spacing between pusher block 112 and knife block 134 can be arranged such that pusher block 112 initially engages knife block 134 after the movement of cam member 152 to its operative position is completed. When pusher block 112 engages knife block 134, the advance of knife blade 138 along central longitudinal slots 42 and 62 of anvil 40 and staple cartridge 60, respectively, is initiated. Thereafter, staple pusher bars 124 and knife blade 138 are advanced simultaneously to staple and cut the tissue gripped between anvil 40 and staple cartridge 60.

As pusher block 112 is advanced, staple pusher bars 124 are moved longitudinally along slots 66 provided in staple cartridge 60. The two wedge-like cam surfaces 130 of staple pusher bars 124 move through slots 66 into engagement with the sloped surfaces of staple drivers 65 to sequentially drive staples 61 from cartridge 60 and to form staple 61 into B-shaped configuration against anvil flanges 38. The cam surfaces 130 are located at the same distance from pusher block 112 to simultaneously actuate staple drivers 65 located on opposite sides of central longitudinal slot 62. At the same time, knife block 134 is advanced to move knife blade 138 through central longitudinal slot 42 of anvil 40 and through central longitudinal slot 62 of staple cartridge 60 to cut the tissue gripped between the jaw members. The additional clamping forces applied to the front portions of upper jaw member 22 and lower jaw member 24 via cam mechanism 150 tend to resist the forces exerted on anvil 40 and staple cartridge 60 when staples 61 are formed.

After pusher block 112 is fully advanced to form all of the staples in cartridge 60, the pusher block is retracted toward its start position by retraction of actuator knob 114. Initially, only pusher block 112 moves backward from staple cartridge 60 because staple pusher bars 124 slide through knife block 134 which remains stationary. When offset portions 142 of staple pusher bars 124 engage the front of knife block 134, the knife block is moved backward from staple cartridge 60 along with pusher block 112. As a result, staple pusher bars 124 and knife blade 138 are simultaneously retracted from staple cartridge 60 and anvil 40.

As pusher block 112 returns toward its start position, cam actuator pin 174 engages sloped surface 166 of rear cam finger 164 to pivot cam member 152 in a clockwise direction toward its inoperative position. Cam actuator pin 174 moves along sloped surface 166 into slot 160 between cam fingers 162 and 164 to return cam member 152 to its inoperative position. As a result, second cam surface 158 of cam member 152 is disengaged from the top wall of upper jaw member 22 and rear end of top wall 31 of upper jaw member 22 moves downward into engagement with first cam surface 156. At the same time, front cam finger 162 pivots downward into gap 172 between fingers 170 on pusher block 112, and both cam fingers 162 and 164 pivot downward into slot 168 formed in bottom wall 53 of lower jaw member 24. Thereafter, with cam member 152 in its inoperative position, latching arm 92 can be pivoted downward, as shown in Figure 2, to permit upper jaw member 22 and lower jaw member 24 to be disassembled. At this point, the cut and stapled tissue can be removed from the jaw members.

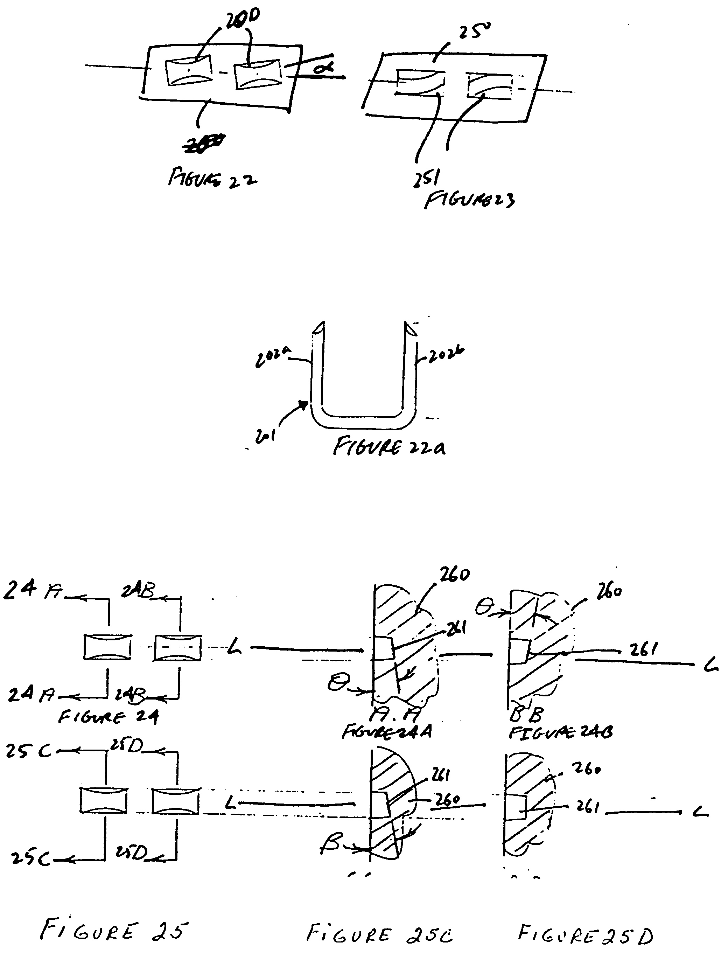

An improvement to the present invention in shown in Figures 22, 23, 24 and 25. As seen in Figures 22 and 23 one of the potential alternatives to aligning the anvil pockets is described therein. As seen in Figure 22, the anvil pockets 200 have been adjusted for a respective staple along the center line of the staple. Now, the anvil pockets 200 are slightly skewed at an angle α to the center line of the staple. For instance, in the staple 201 of Figure 22a, these anvil pockets 200 would engage each of the legs 202a, 202b of a staple. Accordingly, when the staple is crimped, each respective leg 202b of the staple 201 is now skewed with respect to the leg 202a so that when the staple legs 202a, 202b are crimped they do not contact one another with a very high degree of certainty. In contrast, on occasion it would be possible for the staple legs 202a, 202b as shown in Figure 21 to contact one another such that it is difficult to completely form the staple.

An alternative is seen in Figure 23 wherein the base 251 of the anvil 250 is no longer flat but curved to further insure the legs 202a, 202b of the staple do not contact one another upon crimping. Yet another potential embodiment is shown in Figures 24, 24a and 24b as well as Figures 25, 25c and 25d. As seen in the sketch of Figure 24a and 24b, here the bottom 261 of the staple pockets 260 are canted at respected angles ϑ one away from one another. In this way, when the staple 201 is formed, the legs 202a, 202b of the staple miss one another because they are slightly crimped out of the plane of the opposite staple leg. Of course, as seen in Figures 24c and 24d the angle β can be made far shallower than the angle ϑ described in Figure 23. What is important is that the base 261 of the pockets 260 is now no longer perpendicular to the axis L of the pockets 260 but is rather angled or "tilted", so that the legs 202a, 202b of the staple do not contact one another upon crimping.

Naturally, it will be appreciated that the staple formed in the improved pockets will be able to more accurately be formed and crimped. In this fashion, it is an improvement over the stapler disclosed in the embodiment of Figures 1 through 21. Of course, this staple embodiment can be used with other staplers (as previously explained) such as circular staplers and the like. In this fashion, it is believed that the present invention is useful for all sorts of staplers, and is describe by the attached claims.

## Claims

1. In a surgical stapler containing at least one anvil for crimping a surgical staple, said surgical staple containing a pair of legs attached to a crown, the anvil having pockets which crimp the legs of said staple, and the stapler having a driver which forces said staple legs into said pockets, the improvement comprising:
misaligning said pockets so that each of said pockets forms an angle to a center line drawn between the centers of each of said pockets.

2. The stapler of claim 1 wherein said anvil pockets are each straight with respect to the legs of the said staple.

3. The improvement of claim 1 wherein each of said pockets are curved with respect to the legs of said staple.

4. In a surgical stapler containing at least one anvil for crimping a surgical staple, said surgical staple containing a pair of legs attached to a crown, the anvil having pockets which crimp the legs of said staple, and the stapler having a driver which forces said staple legs into said pockets, the improvement comprising:
wherein said anvil pockets are formed with a pair of walls connected by a base, and said base formed at an angle which is not perpendicular to either of said walls.
